# EUROPEAN PATENT SPECIFICATION

(11) **EP 2 284 162 B1**
(45) Date of publication and mention of the grant of the patent: **02.11.2016**
(21) Application number: 10182191.6
(22) Date of filing: 19.05.2006
(51) Int. Cl.: C07D 303/08, C07C 29/62, C07C 31/34, C08G 59/04, C07D 301/26, C07D 301/06, C07C 29/82, B01D 3/14

(54) **Process for producing dichloropropanol**
Verfahren zur Herstellung von Dichloropropanol
Procédé de fabrication de dichloropropanol

(30) Priority: 20.05.2005 FR 0505120; 08.11.2005 US 734659 P; 08.11.2005 US 734627 P; 08.11.2005 US 734657 P; 08.11.2005 US 734658 P; 08.11.2005 US 734635 P; 08.11.2005 US 734634 P; 08.11.2005 US 734637 P; 08.11.2005 US 734636 P; 20.05.2005 EP 05104321
(43) Date of publication of application: 16.02.2011
(62) Divisional of application: 06755263.8
(73) Proprietor: SOLVAY SA, 1120 Bruxelles (BE)
(72) Inventor: Gilbeau, Patrick, 7090 Braine-le-Comte (BE); de Andolenko, Ivan, 39500 Tavaux (FR); Krafft, Philippe, 1640 Rhode Saint Genese (BE); Gielen, Freddy, 1330 Rixensart (BE)
(74) Representative: Vande Gucht, Anne

(56) References cited:
- DE-C- 197 308
- DE-C- 238 341
- GB-A- 984 633
- GB-A- 191 314 767
- FAUCONNIER M A: "PREPARATION DE L'EPICHLORHYDRINE", CONFERENCE PROCEEDINGS ARTICLE, vol. 50, no. 50, 1888, pages 212-214, XP009046846,
- GIBSON G P: "THE PREPARATION, PROPERTIES AND USES OF GLYCEROL DERIVATIVES. Part III. THE CHLOROHYDRINS", CHEMISTRY & INDUSTRY, SOCIETY OF CHEMICAL INDUSTRY. LONDON, GB, 1 January 1931 (1931-01-01), pages 949-970, XP009042263, ISSN: 0009-3068

## Description

The present invention relates to processes for producing dichloropropanol.

It is known that natural petrochemical resources, for example oil or natural gas, that are available on earth are limited. Now, these resources are used for producing fuels and as a starting product for producing a large variety of useful organic compounds such as monomers or reactants for producing plastics, for example, ethylene oxide and chloroethanol (see for example K. Weissermel and H.-J. Arpe in Industrial Organic Chemistry, Third Completely Revised Edition, VCH Editor, 1997, page 149), propylene oxide and monochloropropanol (see for example K. Weissermel and H.-J. Arpe in Industrial Organic Chemistry, Third Completely Revised Edition, VCH Editor, 1997, page 275), epichlorohydrin or dichloropropanol (see, for example, Ullmann's Encyclopedia of Industrial Chemistry, 5. ed., Vol. A9, p. 539-540). Documents Chemistry and Industry, November 20, 1931, Part III, pages 949 to 954, and November 27, 1931, Part III, pages 970 to 975, describes a process for the synthesis of dichloropropanol from glycerol and hydrochloric acid in the presence of acetic acid as acid catalyst. DE 197 308 C discloses a method for the synthesis of mono- and dichlorohydrins from glycerol and gaseous hydrochloric acid in the presence of acetic acid, propionic acid, succinic acid, azelaic acid, cinnamic acid, phenyl acetic acid, etc. as catalysts.

According to known processes for producing dichloropropanol, the product is generally obtained in highly diluted aqueous solution with a titre of 5 to 15 % by weight. It is then particularly expensive to purify it. Moreover, the major isomer obtained according to such processes is 2,3-dichloropropane-1-ol.

It was desirable to find uses and processes making it possible to reduce the consumption of natural petrochemical resources, in particular for the abovementioned uses.

It was also desirable to find processes for re-using by-products of other production processes so as to minimize the overall amount of by-products having to be eliminated or destroyed.

It was also desirable to find processes for minimizing the cost of separation operations linked to highly diluted aqueous solutions.

Consequently, the invention relates to a process for producing dichloropropanol, by subjecting glycerol to a reaction with a chlorinating agent, according to which the glycerol used contains organic impurities selected from fatty acids and esters of fatty acids, the content of said organic impurities being of at most 8 % by weight, wherein the esters of fatty acids are mono esters of glycerol with fatty acids and wherein the fatty acids are selected from saturated and unsaturated fatty acids containing more than 12 carbons atoms.

Examples of esters of glycerol are glycerol monostearates.

Glycerol can be synthetic glycerol, glycerol obtained from renewable raw materials or a mixture thereof. Preferably, glycerol used in the process of the invention has at least partially been produced from renewable raw materials.

The expression « synthetic » means that glycerol has been obtained from fossil raw materials. By fossil raw materials, one intends to denote materials derived from natural petrochemical feedstock, like for instance, petroleum, natural gas, and coal. Among those raw materials, allyl chloride, allyl alcohol and "synthetic" glycerol are more preferred. By "synthetic" glycerol, one intends to denote a glycerol obtained from petrochemical feedstocks.

By renewable raw materials, one intends to denote materials obtained from the treatment of renewable raw materials. Among those materials, natural glycerol is preferred. "Natural" glycerol can be obtained for instance by thermochemical conversion of sugars derived from biomass treatments as described in "Industrial Bioproducts : Today and Tomorrow, Energetics, Incorporated for the U.S. Department of Energy, Office of Energy Efficiency and Renewable Energy, Office of the Biomass Program, July 2003, pages 49, 52 to 56". One process is for example the catalytic hydrogenolysis of sorbitol obtained by thermochemical conversion of glucose. Another process is for example the catalytic hydrogenolysis of xylitol obtained by hydrogenation of xylose. Xylose can for example be obtained by hydrolysis of hemicellulose contained in corn fibers.

The expression "glycerol obtained from renewable raw materials" or "natural glycerol" is intended to denote in particular glycerol obtained in the course of the production of biodiesel, or else glycerol obtained during conversions of fats or oils of plant or animal origin in general, such as saponification, trans-esterification or hydrolysis reactions.

Among oils usable in the process of the invention, one can quote all current oils, like the corn, sunflower, old or new colza, babassu, copra, cabbage tree, palm oils, of ricinus and cotton, groundnut oils, soya, flax and crambe and all oils resulting for example from the plants of sunflower or colza obtained by genetic modification or hybridization. One can even use worn oils of crackling, varied animal oils, like fish oils, tallow, the lard and even of greases of squaring. Among oils used, one can still indicate the oils partially modified for example by polymerization or oligomerization such as for example "linseed oil stand oils", sunflower and puffed up vegetable oil.

A particularly suitable glycerol can be obtained during the conversion of animal fats. Another particularly suitable glycerol can be obtained during the production of biodiesel. Another yet particularly suitable glycerol can be obtained during the conversion of fats or oils of plant or animal origin, by transesterification in the presence of a heterogeneous catalyst, such as described in documents FR 2752242, FR 2869612 and FR 2869613. More specifically, the heterogeneous catalyst is selected from mixed oxides of aluminium and zinc, mixed oxides of zinc and titanium, mixed oxides of zinc, titanium and aluminium, and mixed oxides of bismuth and aluminium, and the heterogeneous catalyst is used in a fixed-bed configuration. In the process according to the invention, glycerol can be as described in the patent application entitled « Process for preparing a chlorohydrin by conversion of multi-hydroxylated aliphatic hydrocarbons" filed in the name of SOLVAY SA on the same day as the present application.

Mention is particularly made of a process for manufacturing dichloropropanol, wherein glycerol, the total metal content of which expressed as elements is higher than or equal to 0.1 µg/kg and lower than or equal to 1 000 mg/kg, is submitted to a reaction with a chlorinating agent.

In the process according to the invention, the glycerol used can be a crude glycerol product or a purified glycerol product. A "crude" glycerol product is a glycerol which has not been submitted to any treatment after its manufacture. A "purifiedglycerol product is a glycerol which has been submitted to at least one treatment after its manufacture. When the glycerol is a crude product obtained from renewable raw materials, it can comprise, for example, water in addition to a metal salt. The metal salt is in particular a metal chloride, which is preferably chosen from NaCl and KCl. The metal salt can also be selected from metal sulphates such as sodium sulphate and potassium sulfate. The glycerol used in the process according to the invention has generally a metal salt content of at least 0.5 % by weight, preferably greater than or equal to approximately 1 % by weight, more preferably greater than or equal to approximately 2 % by weight, most preferably greater than or equal to approximately 3 % by weight. The metal salt content is generally of at most 15 % by weight, preferably less than or equal to 10 % by weight, more preferably less than or equal to approximately 7.5 % by weight and most preferably less than or equal to 5 % by weight.

In the process according to the invention, the crude glycerol product contains mono-esters of glycerolwith fatty acid. Preferred fatty acids are saturated and unsaturated fatty acids containing more than 12 carbon atoms like for instance oleic, linoleic and linolenic acids. Those acids are for instance produced during the conversion of colza oil by saponification, trans-esterification and hydrolysis reactions.

In the process according to the invention, the crude product often, comprises at most 6 % by weight of organic impurities. Preferably, it comprises at most 2 % by weight of organic impurities. Most preferably, it comprises at most 1 % by weight of organic impurities.

It has surprisingly been found that the use of crude product having a high content of organic impurities does not have substantial impact on the reaction underlying the process of the invention. Optional byproducts from the organic impurities can easily be eliminated from the reaction mixture e.g., if applicable, by controlling the purge rate as described in the patent application WO 2005/054167 in the name of SOLVAY SA, from page 17, line 33 to page 18, line 2, from page 24, lines 8 to page 25, line 10.

In the process according to the invention, the crude glycerol product generally comprises at least 40 % by weight of glycerol. Often, the crude product comprises at least 50 % by weight of glycerol. Preferably, it comprises at least 70 % by weight of glycerol. Often, the crude product comprises at most 99 % by weight of glycerol. Typically, it comprises at most 95 % by weight of glycerol.

In the process according to the invention, the crude glycerol product generally comprises at least 5 % by weight of water or, in the absence of other compounds than water glycerol, at least 1 % by weight of water. In the process according to the invention, the crude glycerol product generally comprises at most 50 % by weight of water or, in the absence of other compounds other than water and glycerol at most 60 % by weight of water. Often, the crude glycerol product comprises at most 30 % by weight of water, preferably at most 21 % by weight of water.

In another embodiment, the crude glycerol product comprises at most 89 % by weight of glycerol. In that embodiment, the crude glycerol product comprises at most 85 % by weight of glycerol. In that embodiment, the crude glycerol product comprises generally at least 10 % by weight of water and often at least 14 % by weight of water.

The crude glycerol product has a metal salt content preferably greater than or equal to approximately 1 % by weight and more preferably greater than or equal to approximately 1.5 % by weight. The crude glycerol has a metal salt content, preferably less than or equal to 12 % by weight and more preferably less than or equal to approximately 7.5 % by weight.

The term "dichloropropanol" is intended to mean a mixture of isomers comprising 1,3-dichloropropane-2-ol and 2,3-dichloro-propane-1-ol.

In the process for producing dichloropropanol according to the invention, the chlorinating agent can be hydrogen chloride and/or hydrochloric acidic as disclosed in the patent application WO 2005/054167 of SOLVAY SA, from page 4, line 30 to page 6, line 2. Mention can particularly be made a chlorinating agent which can be gaseous hydrogen chloride, aqueous solution of hydrogen chloride or combination of both. Hydrogen chloride can arise from a pyrolysis process of chlorinated organic compounds as for example, a production of vinyl chloride, a production of of 4,4-methylenediphenyl diisocyanate (MDI) or toluene diisocyanate, or from processes for cleansing metals or by reaction of inorganic acids such as sulphuric acid or phosphoric acid on metal chlorides such as sodium chloride, potassium chloride or calcium chloride.

In the process for producing dichloropropanol according to the invention, the chlorinated agent can be aqueous hydrogen chloride or hydrogen chloride preferentially anhydrous, arising from an installation for producing allyl chloride and/or an installation for producing chloromethanes and/or an installation of chlorinolysis and/or a high temperature oxidation installation as described in patent application entitled « Process for manufacturing a chlorohydrin by reaction between a multi-hydroxylated aliphatic hydrocarbon and a chlorinating agent" filed in the name of SOLVAY SA on the same day as the present application.

Mention is particularly made of a process for manufacturing dichloropropanol from glycerolan ester of glycerol, or a mixture thereof, and a chlorinating agent, this agent containing at least one of the following compounds : nitrogen, oxygen, hydrogen, chlorine, a hydrocarbon, a halogenated organic compound, an oxygenated organic compound and a metal.

Mention is particularly made of a hydrocarbon selected from aromatic hydrocarbons, saturated and unsaturated aliphatic hydrocarbons, or mixtures thereof.

Mention is particularly made of an aliphatic unsaturated hydrocarbon selected from acetylene, ethylene, propylene, butene, propadiene, methylacetylene, and mixtures thereof, of a saturated hydrocarbon selected from methane, ethane, propane, butane and mixture thereof, and of an aromatic hydrocarbon which is benzene.

Mention is particularly made of a halogenated organic compound which is a chlorinated organic compound selected from chloromethanes, chloroethanes, chloropropanes, lchlorobutanes, vinyl chloride, vinylidene chloride, monochloropropenes, le perchloroethylene, trichlorethylene, chlorobutadiene, lchlorobenzènes and mixture thereof.

Mention is particularly made of a halogenated organic compound which is a fluorinated organic compound selected from fluoromethanes, fluoroethanes, vinyl fluoride, vinylidene fluoride and mixtures thereof.

Mention is particularly made an oxygenated organic compound which is selected from alcohols, chloroalcohols, chlorethers and mixtures thereof.

Mention is particularly made of a metal selected from alkaline metals, alkaline-earth metals, iron, nickel, copper, lead, arsenic, cobalt, titanium, cadmium, antimony, mercury, zinc, selenium, aluminium, bismuth and mixtures therof.

Mention is more particularly made of a process in which the chlorinating agent is issued at least partially from a process for manufacturing allyl chloride and/or from a process for manufacturing chloormethanes and/or from a chlorinolysis process and/or from a process for oxidizing chlorinated compounds at a temperature higher than or equal to 800°C.

In a more preferred embodiment, the chlorinating agent does not contain gaseous hydrogen chloride.

The process for producing dichloropropanol according to the invention can be carried out in a reactor as specifically disclosed in the patent application WO 2005/054167 of SOLVAY SA from page 6, lines 3 to 23.

Mention is particularly made of an installation made of, or coated with, materials resisting to chlorinating agents, in particular to hydrogen chloride, under the reaction conditions. Mention is more particularly made of an installation made of enamelled-steel or of tantalum.

The process for producing dichloropropanol according to the invention can be carried out in equipments, made of or coated with, materials that are resistant to chlorinating agents, as described in patent application entitled « Process for manufacturing a chlorohydrin in equipments resisting to corrosion" filed under the name of SOLVAY SA on the same day of the present application,.

Mention is particularly made of a process for manufacturing dichloropropanol comprising a stage in which glycerol, an ester of glycerol or a mixture thereof, is submitted to a reaction with a chlorinating agent containing hydrogen choride and at least one other stage carried out in an equipment, made of or covered with, materials resisting to the chlorinating agent under the conditions of this stage. Mention is more particularly made of metallic materials such as enamelled-steel, gold and tantalum and of non-metallic materials such as high density polyethylene, polypropylene, poly(vinylidene fluoride), polytetrafluoroethylene, perfluoro alkoxyalcanes and poly(perfluoropropylvinylether), polysulfones and polysulfides, graphite et impregnated graphite.

The process for producing dichloropropanol according to the invention can be carried out in a reaction mixture as described in patent application entitled « Continuous process for the manufacture of chlorohydrins" filed under the name of SOLVAY SA on the same day as the present application.

Mention is particularly made of a continuous process for manufacturing dichloropropanol, wherein glycerol an ester of glycerol or a mixture thereof, is submitted to a reaction with a chlorinating agent and an organic acid in a liquid reaction medium which composition at the stationnary state comprises glycerol and esters of glycerol, the sum of the contents of glycerol and esters of glycerol being higher than or equal to 1.1 mol % and lower than or equal to 30 mol %, the percentage being expressed with respect to the organic part of the liquid reaction medium.

The organic part of the liquid reaction medium is defined as the sum of the organic compounds of the liquid reaction medium that is to say compounds which molecule contents at least one carbon atom.

In the process for producing dichloropropanol according to the invention, the reaction between glycerol and the chlorinating agent can be carried out in the presence of a catalyst, as specifically disclosed in the patent application WO 2005/054167 of SOLVAY SA from page 6, line 28 to page 8, line 5. Mention is particularly made of a catalyst which is a carboxylic acid or a carboxylic acid derivative having an atmospheric boiling point of greater than or equal to 200°C, preferably adipic acid or an adipic acid derivative.

In the process for producing dichloropropanol according to the invention, the reaction between glycerol and the chlorinating agent can be carried out at a temperature, a pressure and a residence time as specifically disclosed in the patent application WO 2005/054167 of SOLVAY SA from page 8, line 6 to page 10, line 10.

Mention is particularly made of a temperature of at least 20°C and at most 160°C, of a pressure of at least 0.3 bar and at most 100 bar, and of a residence time of at least 1 h and at most 50 h.

In the process for producing dichloropropanol according to the invention, the reaction between glycerol, and the chlorinating agent can be carried out in a solvent as specifically disclosed in the patent application WO 2005/054167 of SOLVAY SA from page 11, line 12 to 36.

Mention is particularly made of an organic solvent such as a chlorinated organic solvent, an alcohol, a ketone, an ester or an ether, a non-aqueous solvent not miscible with glycerol such as chloroethanol, chloropropanol, chlorpropanediol, dichloropropanol, dioxane, phenol,cresol and mixtures of chloropropanediol and dichloropropanol, or heavy products from the reaction such as oligomers of glycerol at least partially chlorinated and/or esterified.

In the process for producing dichloropropanol according to the invention, the reaction between glycerol, and the chlorinating agent can be carried out in the presence of a liquid phase comprising heavy compounds as described in patent application entitled « Process for manufacturing a chlorohydrin in a liquid phase" filed under the name of SOLVAY SA on the same day as the present application.

Mention is particularly made of a process for manufacturing dichloropropanol in which glycerolan ester of glycerol or a mixture thereof, is submitted to a reaction with a chlorinating agent, in the presence of a liquid phase comprising heavy compounds which boiling temperature under 1 bar of absolute pressure is at least 15°C higher than the boiling point of the chlorohydrin under 1 bar of absolute pressure.

The process for producing dichloropropanol according to the invention can be carried under batch mode or continuous mode. Continuous mode is preferred.

In the process for producing dichloropropanol according to the invention, the reaction between glycerol, and the chlorinating agent is preferably carried out in a liquid reaction medium. The liquid reaction medium can be mono- or multiphases.

The liquid reaction medium is made up of all of the dissolved or dispersed solid compounds, dissolved or dispersed gas, dissolved or dispersed liquids, at the temperature of the reaction.

The reaction medium comprises the reactants, the catalyst, the solvent, the impurities present in the reactants, in the catalyst and in the solvent, the intermediates, the products and the by products of the reaction.

By reactants, one intends to denote glycerol and the chlorinating agent.

Among the impurities present in glycerol one can mention carboxylic acids, carboxylic acid salts, esters of fatty acids with glycerol, esters of fatty acids with alcohols used during trans-esterification, inorganic salts such as for example, alkaline and alkaline-earth chlorides and sulfates.

One can mention among the impurities of glycerol, carboxylic acids, carboxylic acid salts, fatty acid esters such as mono-, di- and triglycerides, esters of fatty acids with alcohols used during trans-esterification, inorganic salts such as for example, alkaline and alkaline-earth chlorides and sulfates.

Among intermediates one can mention, the monochlorohydrin of glycerol and its esters and/or polyesters, esters and/or polyesters of glycerol and esters of dichloropropanol.

The ester of glycerol can then be a reactant, an impurity of glycerolor an intermediate.

By products, one intends to denote dichloropropanol and water. Water can be the water produced by the chlorination reaction and/or water introduced in the process.

Among by-products, one can mention for example glycerol oligomers, partially chlorinated and/or esterified.

Intermediates and by-products can be formed in the various steps of the process, for example, during the manufacture of dichloropropanol or during the separation steps of dichloropropanol.

The liquid reaction medium can then contain glycerol, the chlorination agent dissolved or dispersed in the form of bubbles, the catalyst, the solvent, the impurities present in the reactant, the catalyst and the solvent, such as salts dissolved or solid for instance, intermediates, products and by-products of the reaction.

In the process according to the invention, the separation of dichloropropanol from the other compounds of the reaction medium can be carried out as disclosed in the patent application WO 2005/054167 of SOLVAY SA from page 12, line 1 to page 16, line 35 and at page 18, lines 6 to 13. These other compounds are those already mentioned and comprise non-consumed reactants, impurities present in the reactants, in the catalyst and in the solvent, the catalyst, the solvent, the intermediates, water and the by-products of the reaction.

In the process according to the invention, separation and treatment of the other compounds of the reaction medium can be carried out as described in the patent application WO 2005/054167 of SOLVAY SA from page 18, lines 6 to 13.

Mention is particularly made of a separation by azeotropic distillation of a water/dichloropropanol/chlorinating agent mixture in conditions minimizing losses of the chlorinating agent followed by a separation of the dichloropropanol by decantation.

In the process for manufacturing dichloropropanol according to the invention, the separation of dichloropropanol from the other compounds of the reaction medium can be carried out as described in the patent application entitled « « Process for manufacturing a chlorohydrin » filed under the name of SOLVAY SA, on the same day as the present application.

Mention is particularly made of a process for manufacturing dichloropropanol comprising the following steps : (a) glycerol, is submitted to a reaction with a chlorinating agent and an organic acid in order to obtain a mixture containing dichloropropanol and esters of dichloropropanol, (b) at least a part of the mixture obtained in step (a) is submitted to one or more treatments in steps subsequent to step (a) and (c) glycerolis added to at least one of the steps subsequent to step (a), so that to react at a temperature of at least 20°C, with the dichloropropanol esters in order to form at least partially esters of glycerol.

In the process for manufacturing dichloropropanol according to the invention, the separation of dichloropropanol from the other compounds of the reaction medium can be carried out as described in the patent application entitled « Process for manufacturing a chlorohydrin from a multi-hydroxylated aliphatic hydrocarbon" filed in the name of SOLVAY SA on the same day as the present application.

Mention is particularly made of a process for manufacturing dichloropropanol by reaction between glycerol and a chlorinating agent in a reactor which is fed with one or more liquid flows containing less than 50 % by weight of glycerol with respect to the weight of the totality of the liquid flows introduced in the reactor. Mention is more particularly made of a process comprising the following steps : (a) glycerolis reacted with a chlorinating agent in order to obtain at least one medium containing dichloropropanol, water and the chlorination agent, (b) at least one fraction of the medium obtained in step (a) is withdrawn and (c) the fraction withdrawn at step (b) is submitted to a distillation and/or a stripping operation in which glycerolis added in order to separate from the fraction withdrawn at step (b) a mixture containing water and dichloropropanol exhibiting a chlorinating agent reduced content compared to the chlorinated agent content in the fraction withdrawn at step (b).

In the process for manufacturing dichlorpropanol according to the invention, the separation of dichloropropanol from the other compounds of the reaction medium can be carried out as described in the patent application entitled « Process for converting multi-hydroxylated aliphatic hydrocarbons into chlrohydrins" filed under the name of SOLVAY SA, on the same day of the present application.

Mention is particularly made of a process for manufacturing dichloropropanol comprising the following steps : (a) glycerol is reacted with a chlorinating agent in order to obtain a mixture containing dichloropropanol, dichloropropanol esters and water, (b) at least one fraction of the mixture obtained in step (a) is submitted to a distillation and/or stripping treatment in order to obtain a part concentrated in water, dichloropropanol and dichloropropanol esters, and (c) at least one fraction of the part obtained in step (b) is submitted to a separation operation in the presence of at least one additive so as to obtain a portion concentrated in dichloropropanol and dichloropropanol esters, and which contains less than 40 % by weight of water. The separation operation is more particularly a decantation.

In the process according to the invention, separation and treatment of the other compounds of the reaction medium can be carried out as described in the patent application entitled « Process for manufacturing a chlorohydrin by chlorination of a multi-hydroxylated aliphatic hydrocarbon" filed in the name of SOLVAY SA on the same day as the present application, A preferred treatment can consist of submitting a fraction of the other products to a high temperature oxidation.

Mention is particularly made of a process for manufacturing dichloropropanol comprising the following steps : (a) glycerol the alkaline and/or alkaline-earth metals content of which is lower than or equal to 5 g/kg, is reacted with a chlorinating agent and an organic acid, so as to obtain a mixture containing at least dichloropropanol and by-products, (b) at least one part of the mixture obtained at step (a) is submitted to one or more treatments in steps subsequent to step (a) and (c) at least one step subsequent to step (a) is an oxidation at a temperature higher than or equal to 800°C. Mention is more particularly made of a process in which in the subsequent step, a part of the mixture obtained at step (a) is withdrawn and that part is submitted to an oxidation at a temperature higher than or equal to 800°C, during the withdrawal. Mention is also made of a process in which the treatment of step (b) is a separation operation selected from the operations of decantation, filtration, centrifugation, extraction, washing, evaporation, stripping, distillation, adsorption or the combination of at least two of them.

In the process for producing dichloropropanol according to the invention, vapour stripping, in particular steam stripping of the reaction medium, can be carried out. The reaction medium is defined as above. This medium is preferably a liquid reaction medium (a liquid phase) as defined above. When the reaction medium is a liquid phase, the expression "reaction medium" also includes the gas phase in equilibrium with the liquid. In the following, the expression "reaction medium" will then be used to designate indistinctly the liquid phase where the reaction between glyceroland the chlorinating agent occurs and the gas phase in equilibrium with that liquid phase. When vapour stripping of the reaction medium is carried out, it is possible to obtain a stripped fraction containing from 1 to 5, some times from 2 to 3 and preferably from 1.5 to 2.5 mol/l ofdichloropropanol. The stripped fraction is mainly composed of water and dichloropropanol.

In the process for producing dichloropropanol according to the invention, when dichloropropanol is not completely removed from the reaction mixture by withdrawal of a fraction containing water, it is possible to recover at least another fraction of the reaction mixture containing dichloropropanol.

In this aspect of the process for producing dichloropropanol according to the invention, at least one fraction comprising from 50 to 95 % by weight of dichloropropanol and at most 50 % by weight of water is generally recovered. Preferably, this fraction comprises from 75 to 99.9 %, often from 75 to 99 %, by weight of dichloropropanol and from 0.01 to 25 %, often from 1 to 25 %, by weight of water.

The recovery is preferably carried out by distillation or evaporation. Other fractions obtained during this step, comprising, for example, intermediates and, optionally, glyceroland the catalyst, can be recycled to the reaction with the chlorinating agent. It is also possible to separate at least one fraction containing heavy by-products of the reaction, such as described in the patent application WO 2005/054167 of SOLVAY SA from page 11, line 32 to page 11, line 34, in particular chlorinated polymers of glycerol, which can be destroyed or can optionally be used in a process for producing polymers of glycerol for example by dechlorination.

The distillation or evaporation is generally carried out at a temperature of at least 20°C. This temperature is often at least 60°C. It is preferably at least 70°C. The distillation or evaporation is generally carried out a temperature of at most 180°C. This temperature is preferably at most 140°C.

The distillation or evaporation is generally carried out at a pressure of greater than 0.001 bar. This pressure is preferably greater than or equal to approximately 0.003 bar. The distillation or evaporation is generally carried out at a pressure of at most 15 bar. This pressure is often at most 10 bar. It is preferably at most 7 bar, more preferably at most 1 bar, yet more preferably at most 0.5 bar and most preferably at most 0.1 bar.

The distillation or evaporation operation can be carried out either by means of distillation columns or by means of evaporators, of film evaporators or alternatively of wiped thin film evaporators.

The recoverable fractions of the residues can be separated there from by physical and/or chemical operations. An example of physical operation is a distillation advantageously by means of a wiped thin film evaporator with an interior or exterior condenser. An example of a chemical operation is an hydrolysis of the residue to recover for instance the catalyst.

The process for producing dichloropropanol according to the invention can be carried out, for example, in cascade reactors, in at least one plate column or in at least one bubble column, or an assembly of such reactors.

The reactors may effectively be of a type that is stirred either by means of internal stirring, or by means of a recirculation pipe exterior to the reactor.

When, in the process according to the invention, the reaction medium is heated, the heating can be obtained, for example, by means of a jacket or by means of an internal heat exchanger. Heating can also be obtained by means of a heat exchanger on a recirculation pipe exterior to the reactor. Optionally, the heating is obtained by combined use of a jacket and of a heat exchanger on a recirculation pipe exterior to the reactor.

In particular when the process according to the invention is operated in a continuous or fed-batch mode, secondary reactions can lead to the build-up in the reactor of by-products of low volatility, among which more or less chlorinated oligomers of glycerol. This build-up can lead to a progressive increase of the volume of the reaction medium, to a progressive loss of productivity and require a continuous or discontinuous purge of the reactor to keep the volume at an adequate level. By the expression "purge", one intends to denote a withdrawal of a fraction of the reaction medium.

If appropriate, the catalyst quantity which is removed during such purging operation can be compensated by the introduction of an equivalent quantity of pure or purified catalyst.

The catalyst contained in the purge from the reaction mixture can be economically recycled in the reactor after a purification treatment. For example, catalysts with low solubility in water can be subjected to an acid hydrolysis treatment, preferably carried out at a temperature higher than 30°C, preferably at least 50°C which is followed by a separation step e.g. by decantation, filtration or extraction. It has been found that in the case of adipic acid, an acid hydrolysis of the purge leads after cooling and filtration, to the recovery of crystallised adipic acid of high purity with a good yield.

In particular when the process according to the invention is operated in a continuous or fed-batch mode, metal salts, in particular NaCl, optionally present in the raw materials, for example in glycerol from renewable resources described above, can concentrate in the reactor where the reaction between glycerol and the chlorinating agent is carried out. An increase of metal salt content could possibly lead to a progressive crystallisation of insoluble materials, leading to an increase of the volume of the reaction mixture and to various problems linked to the presence of solid materials such as deposit formation on the reactor walls, on the stirrer and on feed and purge lines and valves. Deposit formation on the reactor wall can reduce the heat transfer efficiency and require an increase amount of energy to maintain the temperature of the reaction mixture. Deposit formation on valves and lines can lead to plugging problems. An increased amount of solid in the reaction mixture can reduce the stirring efficiency and require a higher amount of energy to reach a correct agitation. Increase of metal salt concentration could then require a higher continuous or discontinuous purge rate leading to higher losses of products.

While the presence of metal salt is surprisingly acceptable in the process according to the invention, it may therefore be desirable to remove at least part of the metal salt, in particular NaCl, from the reaction system, e.g. in order to prevent optional accumulation of metal salt in the reaction mixture. Such removal can suitably be carried out by subjecting at least a fraction of the reaction mixture which contains metal salt, solid or dissolved, to a treatment comprising at least one separation operation to remove at least part of the metal salt from said fraction.

The separation operation can be selected from liquid/solid, liquid/liquid, liquid/gas and solid/gas separations.

The liquid/solid separation operation can be selected from decantation, centrifugation, filtration, adsorption and treatment with ion-exchanged resins. The liquid/liquid separation operation can be selected from decantation and centrifugation. The liquid/gas separation operation can be selected from stripping, evaporation and distillation.

Liquid/solid separation operations are preferred, filtration is more preferred and filtration where the metal is removed as a solid is most preferred.

The separation step can then be carried out at any step of the process for producing dichloropropanol as described in the patent application WO 2005/054167 of SOLVAY SA from page 12, line 1 to page 18, line 13, for instance after the chlorination reaction, after the step of removing a mixture of dichloropropanol and water from the reaction mixture, after the recovery of dichloropropanol by distillation or evaporation, after the purge of by-products of reaction or after the treatment for recovering the catalyst from the purge.

In a preferred embodiment, the fraction of the reaction mixture which contains metal salt is obtained from the purge of the reactor where the reaction takes place and is sent to a least one separation unit, where the separation of the metal salt is carried out for example by adsorption, distillation, extraction, decantation, centrifugation, filtration and treatment with ion exchanged resins. A liquid/solid separation unit is preferred and a separation by filtration is more preferred. The separated liquid is preferably recycled back to the reactor and the metal salt is left on the filter.

The filtration step can be carried out at a temperature which is usually greater than or equal to 4 °C, preferably greater than or equal to 20 °C, more preferably greater than or equal to 30 °C, yet more preferably greater than or equal to 50 °C and most preferably greater than or equal to 80 °C. This temperature is generally lower than or equal to 150 °C and preferably lower than or equal to 144 °C.

The nature of the filtration system is not critical and is readily apparent to the skilled person aware of the present invention. A description of suitable filtration systems can be found in "Perry's Chemical Engineers'Handbook, Sixth Edition, 1984, Sections 19-65 to 19-103".

As the metal salt accumulates on the filtration system, it is generally recommended to periodically regenerate the filtration unit by removing the filtrated salt. The regeneration can be performed by any means, for example by removing, in particular by mechanical means, the solid or by dissolving the solid. Optionally, solid elution treatments can be incorporated in the regeneration procedure.

When anhydrous HCl is used as chlorinating agent, it is preferred to direct a liquid stream comprising glycerolagainst the current of the stream of HCl. When the process is carried out in several reactors, the HCl is advantageously dried between two reactors, for example by adsorption on a suitable solid, such as a molecular sieve, or by reverse osmosis through a suitable membrane.

This particular embodiment of the process according to the invention makes it possible to obtain, particularly economically, a concentrated dichloropropanol often having a dichloropropanol content of greater than or equal to 90 % by weight relative to the total weight of the chlorohydrin. By means of this approach, it is possible to obtain 1,3-dichloropropane-2-ol as major isomer with an isomeric purity of greater than 80%.

In the process according to the invention, the mixture can contain the 1,3-dichloropropane-2-ol :and 2,3-dichloropropane-1-ol isomers in a mass ratio 1,3-dichloropropane-2-ol : 2,3-dichloropropane-1-ol generally higher than or equal to 0.5, often higher than or equal to 3, frequently higher than or equal to 7 and in particular higher than or equal to 20.

In the process for producing dichloropropanol according to the invention, the dichloropropanol can contain a high amount of halogenated ketones in particular chloroacetone as described in patent application FR 05.05120 of SOLVAY SA filed on May 20, 2005,. In the process for producing dichloropropanol according to the invention, the halogenated ketone content of the dichloropropanol can be decreased by submitting the dichloropropanol to an azeotropic distillation in the presence of water or by submitting the dichloropropanol to a dehydrochlorination treatment, as described in patent application FR 05.05120 of SOLVAY SA filed on May 20, 2005.

Mention is particularly made of a process for manufacturing epichlorohydrin in which halogenated ketones are formed as by-products and which comprises at least one treatment for the elimination of at least one part of the formed halogenated ketones. Mentions are more particularly made of a process for manufacturing epichlorohydrin by dehydrochlorination of dichloropropanol where at least a fraction of the dichloropropanol is manufactured by chlorination of glycerol, of a treatment of dehydrochlorination and of a treatment by azeotropic distillation of a mixture water-halogenated ketone, both treatments used in order to eliminate at least a part of the formed halogenated ketones and of a process for manufacturing epichlorohydrin in which the halogenated ketone is chloroacetone.

In the process for producing dichloropropanol according to the invention, a high selectivity for 1,3-dichloropropane-2-ol is surprisingly obtained, which isomer is particularly suitable as starting product for a dehydrochlorination with a view to producing epichlorohydrin.

In the process according to the invention, dichloropropanol can be submitted to a dehydrochlorination reaction to produce epichlorohydrin, as described in patent applications WO 2005/054167 and FR 05.05120 in the name of SOLVAY SA.

In the process according to the invention, dichloropropanol can be submitted to a dehydrochlorination reaction as described in patent application entitled « Process for manufacturing an epoxide from a multi-hydroxylated aliphatic hydrocarbon and a chlorinating agent" filed in the name of SOLVAY SA on the same day as the present application.

Mention is particularly made of a process for manufacturing epichlorohydrin wherein a reaction mixture resulting from the reaction of glyceroland a chlorinating agent, the reaction mixture containing less than 10 g of dichloropropanol per kg of the reaction mixture, is submitted to a further chemical reaction without intermediate treatment.

Mention is particularly made of a process for the manufacture of epichlorohydrin comprising the following steps : (a) glycerolis submitted to a reaction with a chlorinating agent and an organic acid in order to form dichloropropanol and esters of dichloropropanol, in a reaction mixture containing, glycerol, esters glycerol, water, the chlorinating agent and the organic acid, the reaction mixture containing at least 10 g of dichloropropanol per kg of the reaction mixture (b) at least one fraction of the mixture obtained in step, fraction which has the same composition as the reaction mixture obtained at step (a), is submitted to one or more treatment in steps subsequent to step (a), and (c) a basic compound is added at least one of the step subsequent to step (a) so as it reacts at least partially with the dichloropropanol, the esters of the dichloropropanol, the chlorinating agent and the organic acid in order to form epichlorohydrin and salts.

The process for producing dichloropropanol according to the invention can be integrated in a global scheme such as described in patent application entitled « Process for manufacturing an epoxide from a chlorohydrin" filed in the name of SOLVAY SA on the same day as the present application.

Mention is particularly made of a process for manufacturing epichlorohdyrin comprising at least one purification step of the formed epichlorohydrin, the epichlorohydrin being at least partially manufactured by a process of dehydrochlorination of dichloropropanol, the dichloropropanol being at least partially manufactured by a process of chlorination of glycerol.

The process of the invention can be followed by a manufacture of epichlorohydrin by dehydrochlorination of dichloropropanol and epichlorohydrin can usefully be used for manufacturing epoxy resins.

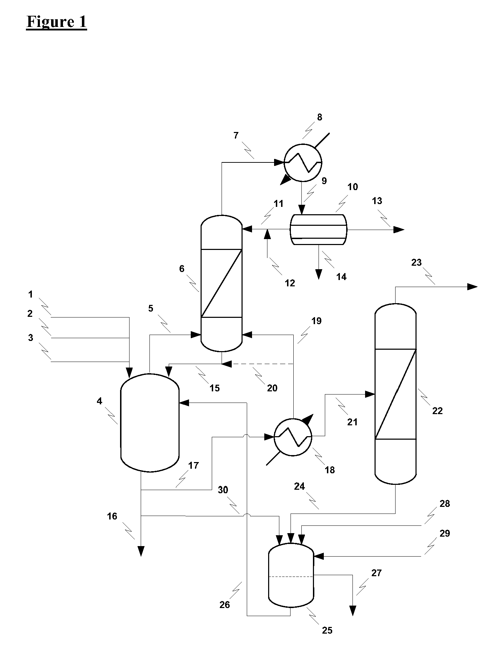

Figure 1 shows a preferred particular scheme for a plant that can be used for carrying out the process for producing dichloropropanol according to the invention : A reactor (4) is fed, in a continuous or batch mode, with glycerol, via line (1) and catalyst via line (2), the feed of the chlorinating agent, is carried out continuously or in batch-mode via line (3), a distillation column (6) is fed via line (5) with vapour produced from reactor (4), a stream is withdrawn from column (6) via line (7) and fed to a condenser (8), the stream from the condenser is fed via line (9) to a decanter (10) in which aqueous and organic phases are separated. A fraction of the separated aqueous phase is optionally recycled via line (11) to the top of the column for maintaining reflux. Fresh water can be added via line (12) to the top of the column for maintaining reflux. The production of dichloropropanol is distributed between the organic phase withdrawn through line (14) and the aqueous phase withdrawn through line (13). The residue from column (6) can be recycled to the reactor via line (15). Heavy by-products can optionally be removed from the reactor by means of a purge (16) located in the liquid bottom of the reactor. A stream is withdrawn from the purge (16) and fed via line (17) into an evaporator (18) wherein a partial evaporation operation is carried out e.g. by heating or by gas sweeping with nitrogen or steam, the gas phase containing most of the chlorinating agent from stream (17) is recycled via line (19) to the column (6) or via line (20) to the reactor (4), a distillation column or stripping column (22) is fed with the liquid phase arising from the evaporator (18) via line (21), the main fraction of the chlorohydrin is collected from the top of the column (22) through line (23) and the column residue is fed via line (24) to a filtration unit (25) in which solid and liquid phases are separated, the liquid phase is recycled via line (26) to the reactor (4). The solid can be withdrawn from the filtration unit (25) via line (27) as a solid or as a solution. Solvents can be added to the filtration unit (25) via lines (28) and (29) for washing and/or dissolution of the solid and withdrawn from line (27). Optionally, a stream is withdrawn from the purge (16) and fed via line (30) into a filtration column (25). The stripper (18) and the distillation column (22) are then bypassed.

Results obtained according to this last scheme (stripper (18) and column (22) bypassed) are detailed in example 1.

This variant of the process allows to remove at the top by azeotropy almost all of the water arising from the reaction, from the starting materials and/or possibly fed in the bottom of the reactor or of the column and to obtain a mixture of dichloropropanols of very high purity, above 99.5 % by weight for the sum of the two isomers, with a selectivity related to hydrocarbon chain and hydrogen chloride higher than 99 % by weight and to remove the metal salt which can build up in the reactor when crude glycerol is used in the reaction.

The example below are intended to illustrate the invention without, however, limiting it.

### Example 1

The numbers in parentheses refer to Figure 1. The additional equipment in the schema of figure 1, with stripper (18) and column (22) has not been used in this case.

Reactor (4) has been continuously fed with crude glycerol and a 33 % by weight hydrochloric aqueous acid solution with relative flow rates mass ratios of 2.06. The crude glycerol was a by product of the biodiesel production and contained 85 % of glycerol, 6 % of NaCl and 0.5 % of organic impurities (fatty acids and derivatives). The residence time was 16 h, the adipic acid concentration in the reaction medium was 2.5 mol of acid functionalities/kg. The reactor has been operated at atmospheric pressure and at 115°C. The reaction mixture has been stripped with of nitrogen and the generated vapor phase has been treated in the distillation column (6) via line (5) (figure 1). The gas phase removed from column (6) has been condensed at 25°C (8) and decanted in the decanter (10). Reflux ratio was adjusted to withdraw the entire production of dichloropropanol at the top of column by recycling an appropriate amount of the aqueous phase from the decantor. At the outlet of the decantor an aqueous phase containing 15.0 % of dichloropropanol (13) and an organic phase (14) containing 88 % of dichloropropanol were recovered. The profiles in organic impurities in these phases were not different from those observed when pure glycerol is used in the process.

A slurry from the reactor has been pumped on a 115 micrometer PTFE membrane filter in the filtration column (25). The salt cake in the filter has been washed at 20°C with dichloropropanol saturated with water. After removal of the liquid phase and draining of the solid, the salt has been dissolved in water and the salted water phase has been discarded. The duration of washing and salt dissolution was about 2 hours. A new filtration cycle of the slurry from the reactor has then been operated. The dichloropropanol washing has been recycled to the reactor by continuous feeding. The analysis of the water phase with salt indicated a dichloropropanol : NaCl mass ratio of 1.44 and a small amount of catalyst (less than 10 g/kg). The quantity of dichloropropanol in the salted water represented 1.6 % of the dichloropropanol total production.

The global yield in dichloropropanol was 93 %.

## Claims

1. Process for producing dichloropropanol, by subjecting glycerol to a reaction with a chlorinating agent, according to which the glycerol used contains organic impurities selected from fatty acids and esters of fatty acids, the content of said organic impurities being of at most 8 % by weight, wherein the esters of fatty acids are mono esters of glycerol with fatty acids and wherein the fatty acids are selected from saturated and unsaturated fatty acids containing more than 12 carbons atoms.

2. Process according to claim 1 wherein the content of said organic impurities is of at most 6 % by weight.

3. Process according to claim 2 wherein the content of said organic impurities is of at most 1 % by weight.

4. Process according to any of claims 1 to 3, wherein the fatty acids are selected from oleic, linoleic and linolenic acids.

5. Process according to any of claims 1 to 4 wherein the monoesters of glycerol are glycerol mono stearates.

6. Process according to any of claims 1 to 5 wherein the glycerol used has been partially obtained during the course of production of biodiesel, or obtained during conversions of fats or oils of plant or animal origin, such conversions being selected among saponification, trans-esterification or hydrolysis reactions.

7. Process according to claim 6 wherein the oil or the grease is selected from corn, sunflower, old or new colza, babassu, copra, cabbage tree, palm oils, oils of ricinus and cotton, groundnut oils, soya, flax and crambe, oils resulting from the plants of sunflower or colza obtained by genetic modification or hybridization, worn oils of crackling, fish oils, tallow, the lard, greases of squaring, "linseed oil stand oils", partially polymerized or oligomerized sunflower and puffed up vegetable oil.

8. Process according to any of claims 1 to 7 wherein the chlorinating agent is gaseous hydrogen chloride.

9. Process according to any of claims 1 to 8 wherein the reaction is carried out in the presence of a catalyst.

10. Process according to claim 9 wherein the catalyst is a carboxylic acid or a carboxylic acid derivative having an atmospheric boiling point of greater than or equal to 200°C and is preferably adipic acid or an adipic acid derivative.

11. Process according to any of claims 1 to 10 followed by a manufacture of epichlorohydrin by dehydrochlorination of dichloropropanol.

12. Process according to claim 11 wherein the epichlorohydrin is used for manufacturing epoxy resins.

## Patentansprüche

1. Verfahren zur Herstellung von Dichlorpropanol durch Umsetzen von Glycerin mit einem Chlorierungsmittel, gemäß dem das verwendete Glycerin aus Fettsäuren und Fettsäureestern ausgewählte organische Verunreinigungen enthält, wobei der Gehalt der organischen Verunreinigungen höchstens 8 Gew.-% beträgt, wobei es sich bei den Fettsäureestern um Monoester von Glycerin mit Fettsäuren handelt und wobei die Fettsäuren aus gesättigten und ungesättigten Fettsäuren mit mehr als 12 Kohlenstoffatomen ausgewählt sind.

2. Verfahren nach Anspruch 1, bei dem der Gehalt der organischen Verunreinigungen höchstens 6 Gew.-% beträgt.

3. Verfahren nach Anspruch 2, bei dem der Gehalt der organischen Verunreinigungen höchstens 1 Gew.-% beträgt.

4. Verfahren nach einem der Ansprüche 1 bis 3, bei dem die Fettsäuren aus Ölsäure, Linolsäure und Linolensäure ausgewählt sind.

5. Verfahren nach einem der Ansprüche 1 bis 4, bei dem es sich bei den Monoestern von Glycerin um Glycerinmonostearate handelt.

6. Verfahren nach einem der Ansprüche 1 bis 5, bei dem das verwendete Glycerin teilweise bei der Herstellung von Biodiesel oder bei Umwandlungen von Fetten oder Ölen pflanzlicher oder tierischer Herkunft erhalten wurden, wobei derartige Umwandlungen aus Verseifungs-, Umesterungs- oder Hydrolysereaktionen ausgewählt sind.

7. Verfahren nach Anspruch 6, bei dem das Öl oder das Fett aus Mais-, Sonnenblumen-, altem oder neuem Kolza-, Babassu-, Kopra-, Oscherstrauch-, Palmölen, Ölen von Rizinus und Baumwolle, Erdnussölen, Soja, Flachs und Krambe, Ölen aus den durch genetische Modifizierung oder Hybridisierung erhaltenen Pflanzen von Sonnenblume oder Kolza, Altbratölen, Fischölen, Talg, Schmalz, Quadraturfetten, Leinölstandölen, teilweise polymerisiertem oder oligomerisiertem Sonnenblumenöl und aufgeblasenem Pflanzenöl ausgewählt ist.

8. Verfahren nach einem der Ansprüche 1 bis 7, bei dem es sich bei dem Chlorierungsmittel um gasförmigen Chlorwasserstoff handelt.

9. Verfahren nach einem der Ansprüche 1 bis 8, bei dem die Umsetzung in Gegenwart eines Katalysators durchgeführt wird.

10. Verfahren nach Anspruch 9, bei dem es sich bei dem Katalysator um eine Carbonsäure oder ein Carbonsäurederivat mit einem Siedepunkt bei Normaldruck größer gleich 200°C und vorzugsweise um Adipinsäure oder ein Adipinsäurederivat handelt.

11. Verfahren nach einem der Ansprüche 1 bis 10, gefolgt von einer Herstellung von Epichlorhydrin durch Dehydrochlorierung von Dichlorpropanol.

12. Verfahren nach Anspruch 11, bei dem das Epichlorhydrin zur Herstellung von Epoxidharzen verwendet wird.

## Revendications

1. Procédé de production de dichloropropanol, par la soumission de glycérol à une réaction avec un agent de chloration, selon lequel le glycérol utilisé contient des impuretés organiques choisies parmi les acides gras et les esters d'acides gras, la teneur desdites impuretés organiques étant d'au plus 8% en poids, où les esters d'acides gras sont des monoesters de glycérol avec des acides gras et où les acides gras sont choisis parmi les acides gras saturés et insaturés contenant plus de 12 atomes de carbone.

2. Procédé selon la revendication 1, dans lequel la teneur desdites impuretés organiques est d'au plus 6% en poids.

3. Procédé selon la revendication 2, dans lequel la teneur desdites impuretés organiques est d'au plus 1% en poids.

4. Procédé selon l'une quelconque des revendications 1 à 3, dans lequel les acides gras sont choisis parmi les acides oléique, linoléique et linolénique.

5. Procédé selon l'une quelconque des revendications 1 à 4, dans lequel les monoesters de glycérol sont des monostéarates de glycérol.

6. Procédé selon l'une quelconque des revendications 1 à 5, dans lequel le glycérol utilisé a été partiellement obtenu au cours de la production de biodiesel, ou obtenu lors des conversions de matières grasses ou d'huiles d'origine végétale ou animale, de telles conversions étant choisies parmi les réactions de saponification, de transestérification ou d'hydrolyse.

7. Procédé selon la revendication 6, dans lequel l'huile ou la matière grasse est choisie parmi les huiles de maïs, de tournesol, de colza ancien ou nouveau, de babassu, de coprah, de sabal, de palme, les huiles de ricin et de coton, les huiles d'arachide, le soja, le lin et le crambe, les huiles résultant des plantes de tournesol ou de colza obtenues par modification génétique ou croisement, les huiles usées issues de craquage, les huiles de poisson, le suif, le lard, les graisses d'équarrissage, les « standolies d'huile de lin », le tournesol partiellement polymérisé ou oligomérisé et l'huile végétale gonflée.

8. Procédé selon l'une quelconque des revendications 1 à 7, dans lequel l'agent de chloration est le chlorure d'hydrogène gazeux.

9. Procédé selon l'une quelconque des revendications 1 à 8, dans lequel la réaction est effectuée en présence d'un catalyseur.

10. Procédé selon la revendication 9, dans lequel le catalyseur est un acide carboxylique ou un dérivé d'acide carboxylique ayant un point d'ébullition atmosphérique supérieur ou égal à 200°C et il s'agit de préférence de l'acide adipique ou d'un dérivé d'acide adipique.

11. Procédé selon l'une quelconque des revendications 1 à 10, suivi de la fabrication d'épichlorhydrine par déshydrochloration de dichloropropanol.

12. Procédé selon la revendication 11, dans lequel l'épichlorhydrine est utilisée pour la fabrication de résines époxy.
